# FASCICULE DE BREVET EUROPEEN

(11) **EP 0 790 254 B1**
(45) Date de publication et mention de la délivrance du brevet: **22.08.2001**
(21) Numéro de dépôt: 97400289.1
(22) Date de dépôt: 10.02.1997
(51) Int. Cl.: C07K 5/023, A61K 38/06

(54) **Pseudopeptides dérivés de neurokinines, leur procédé de préparation et les compositions pharmaceutiques qui les contiennent**
Von Neurokininen abgeleitete Pseudopeptide, Verfahren für ihre Herstellung und sie enthaltende pharmazeutische Zubereitungen
Pseudopeptides derived from neurokinines, process of preparation and pharmaceutical compositions comprising them

(30) Priorité: 16.02.1996 FR 9601919
(43) Date de publication de la demande: 20.08.1997
(73) Titulaire: ADIR ET COMPAGNIE, 92415 Courbevoie Cédex (FR)
(72) Inventeur: Fauchere, Jean-Luc, 92210 Saint-Cloud (FR); Kucharczyk-Gentric, Nathalie, 92130 Issy Les Moulineaux (FR); Canet, Emmanuel, 75014 Paris (FR); Lonchampt, Michel, 94150 Chevilly La Rue (FR)

(56) Documents cités:
- WO-A-94/05693
- FR-A- 2 719 312
- EUROPEAN JOURNAL OF PHARMACOLOGY, vol. 310, 1996, pages 37-46, XP000674856 J. BONNET ET AL.: "A water-soluble, stable dipeptide NK1 receptor-selective neurokinin receptor antagonist with potent in vivo pharmacological effects: S18523"

## Description

La présente invention concerne de nouveaux pseudopeptides dérivés de neurokinines, leur procédé de préparation et les compositions pharmaceutiques qui les contiennent.

Les neurokinines forment une famille de neuropeptides possédant à la partie C terminale une analogie de structure : Phe-X-Gly-Leu-Met-NH₂. Ces neuropeptides, substance P (SP), neurokinine A (NKA) et neurokinine B (NKB) induisent une contraction rapide des fibres musculaires lisses par opposition aux contractions lentes développées par la bradykinine. Largement représentées au niveau du corps humain, en particulier au niveau du système nerveux central et du système nerveux périphérique, leurs effets agonistes endogènes s'effectuent via des récepteurs spécifiques avec une affinité préférentielle de SP, NKA et NKB pour les récepteurs NK₁, NK₂, NK₃. Ils sont impliqués dans de nombreux processus physiologiques ou physiopathologiques tels que nociception, vasoperméabilité, contractions des fibres musculaires lisses, hypersécrétions, et immuno-modulations (OTSUKA M. et coll., Physiol. Rev. 73, 229-308, 1993). De nombreux peptides antagonistes des neurokinines ont été décrits dans la littérature. C'est le cas par exemple des composés décrits dans les brevets EP 333174, EP 394989, WO 9222569 ou EP 684 257.

La présente invention a pour objet des pseudopeptides synthétiques, qui, outre le fait qu'ils soient nouveaux, se sont montrés particulièrement intéressants par l'intensité de leurs propriétés pharmacologiques. Ils possèdent des propriétés antagonistes sélectives et puissantes vis-à-vis des récepteurs des neurokinines et plus particulièrement des récepteurs NK₁. Ces propriétés les rendent utilisables notamment dans le traitement de la douleur, des processus inflammatoires d'origines diverses, des troubles gastro-intestinaux, de l'asthme, des bronchopathies chroniques, des allergies, des troubles urologiques, de la migraine, des maladies du système nerveux central ainsi qu'en tant qu'antiémétiques.

L'invention concerne plus particulièrement les composés de formule (I): 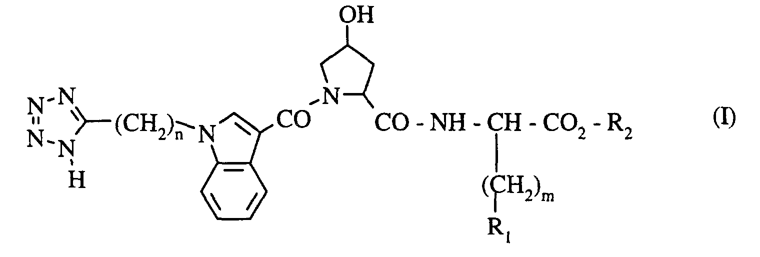 dans laquelle :
- R₁: représente un groupement indolyle, naphtyle, tétrahydronaphtyle, thiényle, phényle (éventuellement substitué par un ou plusieurs atomes d'halogène ou groupements alkyle (C₁-C₆) linéaire ou ramifié, alkoxy (C₁-C₆) linéaire ou ramifié, hydroxy, trihalogénométhyle) ou cycloalkyle (C₃-C₇),
- n: représente un entier tel que 1 ≤ n ≤ 8,
- m: représente un entier tel que 1 ≤ m ≤ 4,
- R₂: représente un groupement benzyle (substitué ou non par un ou plusieurs atomes d'halogène ou groupements alkyle (C₁-C₆) linéaire ou ramifié, alkoxy (C₁-C₆) linéaire ou ramifié, hydroxy ou trihalogénométhyle), un groupement phényle (substitué ou non par un ou plusieurs atomes d'halogène ou groupements alkyle (C₁-C₆) linéaire ou ramifié, alkoxy (C₁-C₆) linéaire ou ramifié, hydroxy ou trihalogénométhyle), un groupement alkyle (C₁-C₆) linéaire ou ramifié,
leurs énantiomères, diastéréoisomères et épimères ainsi que leurs sels d'addition à un acide ou à une base pharmaceutiquement acceptable.

Parmi les acides pharmaceutiquement acceptables, on peut citer à titre non limitatif les acides chlorhydrique, bromhydrique, sulfurique, phosphonique, acétique, trifluoroacétique, lactique, pyruvique, malonique, succinique, glutarique, fumarique, tartrique, maléïque, citrique, ascorbique, oxalique, méthane sulfonique, camphorique, etc...

Parmi les bases pharmaceutiquement acceptables, on peut citer à titre non limitatif l'hydroxyde de sodium, l'hydroxyde de potassium, la triéthylamine, la tertbutylamine, etc...

Les composés préférés de l'invention sont ceux pour lesquels R₁ représente un groupement indolyle.

L'invention s'étend également au procédé de préparation des composés de formule (I). L'un de ces procédés est caractérisé en ce que l'on fait réagir une hydroxyproline protégée, de formule (II), dont on a éventuellement séparé les isomères : 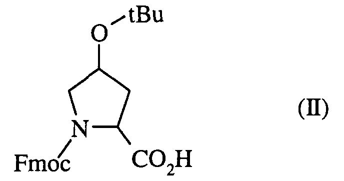 dans laquelle :
Fmoc représente le groupement 9-fluorénylméthoxycarbonyle,
tBu représente le groupement tert-butyle,
avec de l'alcool benzylique, en présence de 4-diméthylaminopyridine et de N,N-dicyclohexylcarbodiimide,
pour conduire au composé de formule (III) : 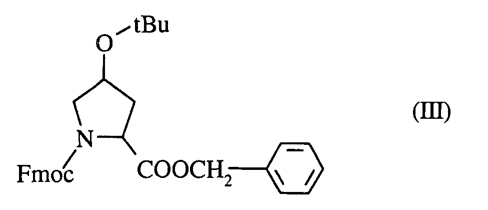 dans laquelle Fmoc et tBu ont les mêmes significations que précédemment,
dont on déprotège la fonction amine en milieu pipéridine,
pour conduire au composé de formule (IV) : 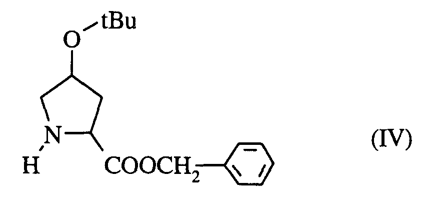 dans laquelle tBu a la même signification que précédemment,
que l'on fait réagir avec de l'acide indole-3-carboxylique,
pour conduire au composé de formule (V) : 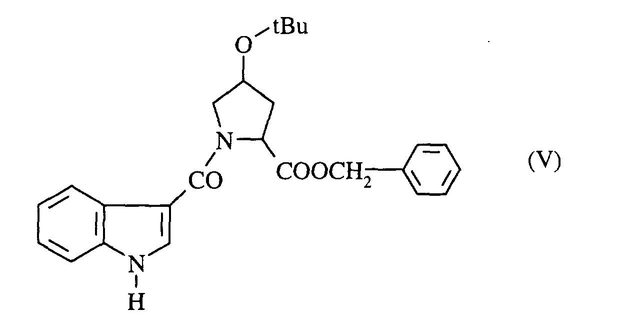 dans laquelle tBu a la même signification que précédemment,
dont on déprotège la fonction acide carboxylique, par hydrogénation catalytique,
pour conduire au composé de formule (VI) : 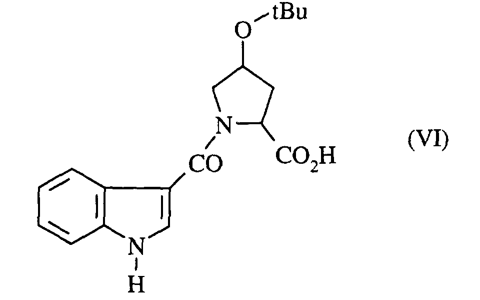 dans laquelle tBu a la même signification que précédemment,
que l'on met en réaction avec un amino-acide protégé de formule (VII), dont on a éventuellement séparé les isomères : 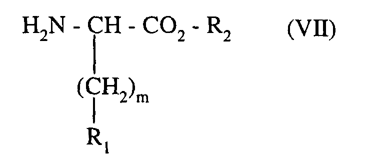 dans laquelle R₁, R₂ et m ont la même signification que dans la formule (I),
pour conduire au composé de formule (VIII) : 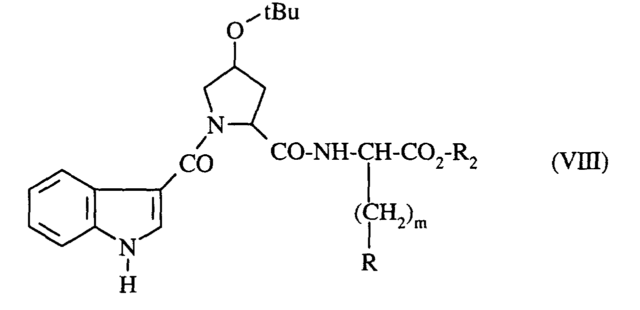 dans laquelle tBu, R₁, R₂ et m ont la même signification que précédemment,
que l'on fait réagir sur un tétrazole protégé de formule (IX), en présence d'hydrogénosulfate de tétrabutylammonium, 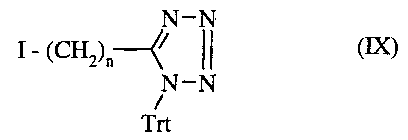 dans laquelle Trt représente le groupement triphénylméthyle,
pour conduire au composé de formule (X) : 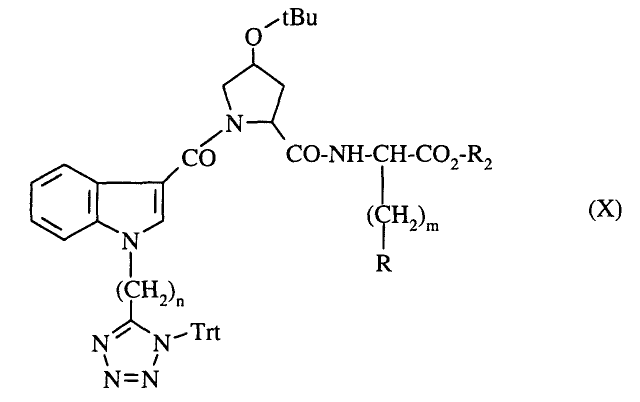 dans laquelle tBu, R₁, R₂, m, n et Trt ont la même signification que précédemment,
que l'on déprotège, en milieu acide trifluoroacétique,
pour conduire au composé de formule (I),
- qui peut être, le cas échéant, purifié selon une technique classique de purification,
- dont on sépare, le cas échéant, les isomères selon une technique classique de séparation,
- que l'on transforme, si on le souhaite, en ses sels d'addition à une base pharmaceutiquement acceptable.

Le composé de formule (IX) est obtenu par réaction d'un nitrile de formule (XI) :

Cl - (CH₂)ₙ-CN (XI)

dans laquelle n a la même signification que dans la formule (I),
avec de l'azoture de sodium en présence de chlorure d'aluminium, sous atmosphère inerte, pour conduire au composé de formule (XII) : 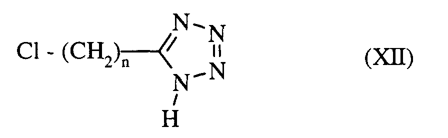 dans laquelle n a la même signification que dans la formule (I),
que l'on fait réagir avec le chlorure de triphénylméthyle en présence de triéthylamine, pour conduire au composé de formule (XIII) : 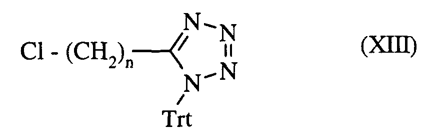 dans laquelle n et Trt ont la même signification que précédemment,
qui est ensuite traité avec de l'iodure de sodium, pour conduire au dérivé iodé de formule (IX) correspondant.

Un autre procédé de préparation des composés de formule (I) est caractérisé en ce que l'on fait réagir l'ester benzylique de l'acide indole-3-carboxylique avec un bromonitrile de formule (XIV) en présence d'hydrure de sodium :

Br - (CH₂)ₙ - CN (XIV)

dans laquelle n a la même signification que dans la formule (I),
pour conduire au composé de formule (XV) : 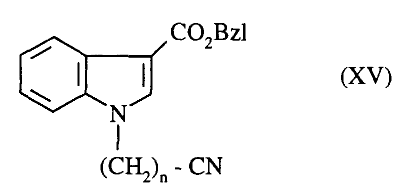 dans laquelle n a la même signification que dans la formule (I) et Bzl représente un groupement benzyle, dont on déprotège la fonction acide par hydrogénation catalytique,
pour conduire au composé de formule (XVI) : 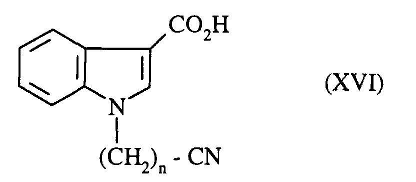 dans laquelle n a la même signification que dans la formule (I), que l'on fait réagir avec une hydroxyproline protégée de formule (XVII), en présence de bromo-tris-pyrrolidino-phosphonium hexafluorophosphate et de diisopropyléthylamine : 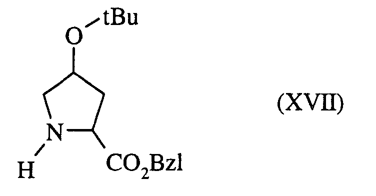 dans laquelle tBu représente le groupement tert-butyle et Bzl le groupement benzyle,
pour conduire au composé de formule (XVIII): 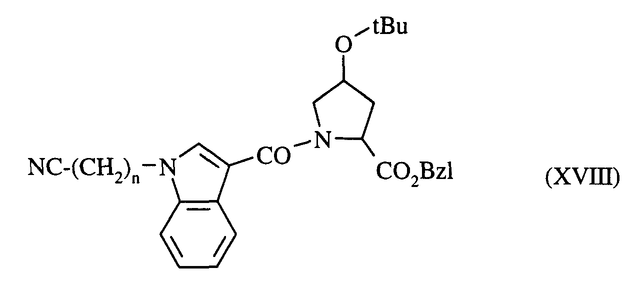 dans laquelle n, tBu et Bzl ont la même signification que précédemment, dont on déprotège la fonction acide en milieu basique en présence d'un catalyseur,
pour conduire au composé de formule (XIX): 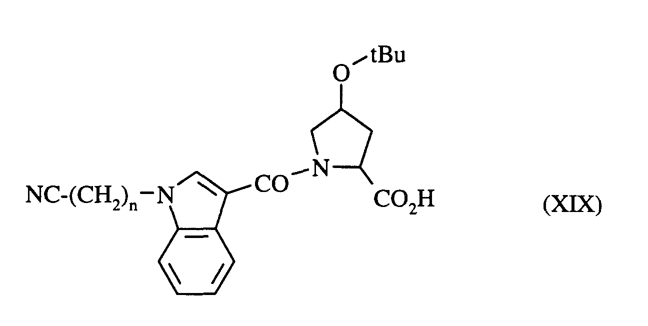 dans laquelle n et tBu ont la même signification que précédemment, que l'on fait réagir avec un composé de formule (XX) en présence d'un réactif de couplage peptidique : 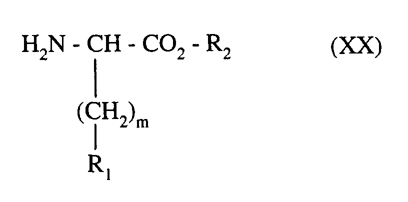 dans laquelle R₁, R₂ et m ont la même signification que dans la formule (I),
pour conduire au composé de formule (XXI) : 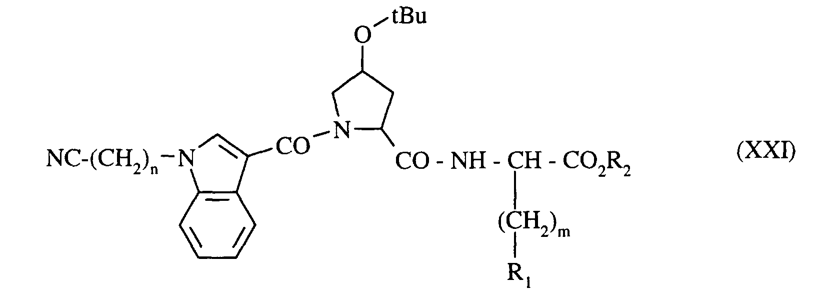 dans laquelle R₁, R₂, n, m et tBu ont la même signification que précédemment, que l'on traite ensuite avec du triméthylazidosilane et de l'oxyde de dibutylétain,
pour conduire au composé de formule (XXII) : 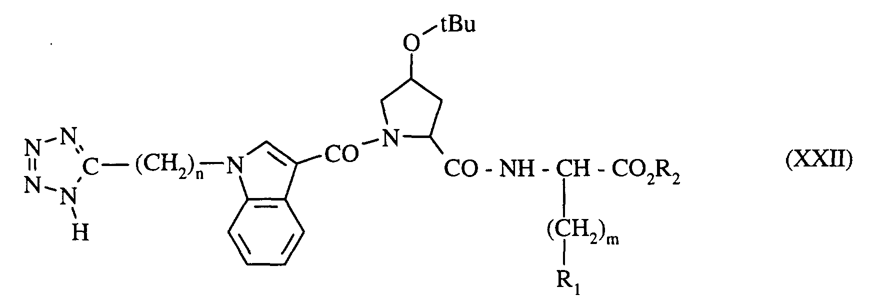 dans laquelle R₁, R₂, n, m et tBu ont la même signification que précédemment, que l'on déprotège en milieu acide trifluoroacétique, pour conduire au composé de formule (I),
- qui peut être, le cas échéant, purifié selon une technique classique de purification,
- dont on sépare, le cas échéant, les isomères selon une technique classique de séparation,
- que l'on transforme, si on le souhaite, en ses sels d'addition à une base pharmaceutiquement acceptable.

Les composés de l'invention possèdent des propriétés pharmacologiques très intéressantes. Ce sont des ligands spécifiques des récepteurs des neurokinines qui possèdent en particulier des propriétés antagonistes particulièrement intenses vis-à-vis des récepteurs NK₁. Les récepteurs NK₁ seraient plus particulièrement impliqués dans la régulation de la transmission de la douleur, de l'oedème induit par augmentation de la vasoperméabilité, des phénomènes secrétoires au niveau trachéo-bronchique et gastro-intestinal, de la salivation, du contrôle ventilatoire et du tonus vasculaire, et de l'activation des cellules participant aux processus inflammatoires. De plus, contrairement à certains dérivés pseudopeptidiques antagonistes de substance P, ces composés sont dépourvus d'effet dégranulant sur les cellules mastocytaires.

D'autre part, outre le fait qu'ils soient nouveaux, les composés de la présente invention se sont révélés particulièrement puissants, vis-à-vis en particulier des composés les plus proches de l'art antérieur décrits dans le brevet EP 684 257. Les modifications structurales ne pouvaient en aucun cas laisser prévoir ce gain d'activité.

La présente invention a également pour objet les compositions pharmaceutiques renfermant comme principe actif au moins un composé de formule (I) seul ou en combinaison avec un ou plusieurs excipients ou véhicules inertes non toxiques.

Parmi les compositions pharmaceutiques selon l'invention, on pourra citer plus particulièrement celles qui conviennent pour l'administration orale, parentérale, nasale, les comprimés simples ou dragéifiés, les comprimés sublinguaux, les gélules, les tablettes, les suppositoires, les crèmes, pommades, gels dermiques, les préparations injectables, etc...

La posologie utile varie selon l'âge et le poids du patient, la nature et la sévérité de l'affection ainsi que la voie d'administration. Celle-ci peut être orale, nasale, rectale ou parentérale. D'une manière générale, la posologie unitaire s'échelonne entre 0,2 et 100 mg pour un traitement en 1 à 3 prises par 24 heures.

Les exemples suivants illustrent l'invention et ne la limitent en aucune façon.

Les produits de départ utilisés sont des produits connus ou préparés selon des modes opératoires connus.

Dans les exemples ci-dessous, les acides aminés dont les abréviations commencent par une lettre majuscule sont de configuration L. Les acides aminés dont les abréviations commencent par une lettre minuscule sont de configuration D.

Les structures des composés de l'invention ont été confirmées par les techniques spectroscopiques usuelles (résonance magnétique nucléaire, infrarouge, spectrométrie de masse, ...).

Les abréviations utilisées dans les exemples sont les suivantes :
**Hyp** représente le résidu **(R)-4-hydroxy-L-prolyle** de formule : 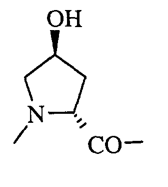
**Trp-OBzl(3,5-ditrifluorométhyl)** représente le résidu de formule : 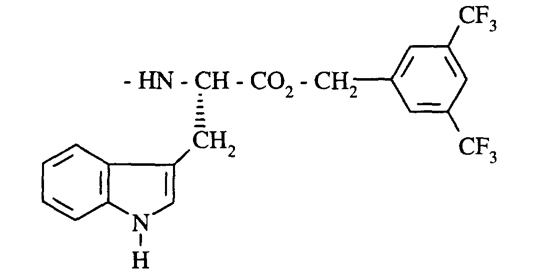
**tBu** représente le groupement **tert-butyle**,
**Bzl** représente le groupement **benzyle**,
**H-Hyp(tBu)-OBzI** représente le résidu de formule : 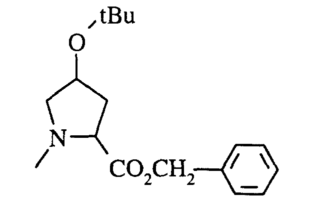
**Nal-OBzl(3,5-ditrifluorométhyl)** représente le résidu de formule : 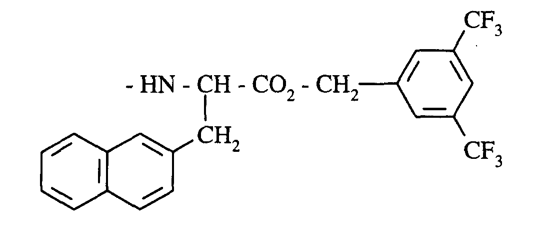
**Tna-OBzI** représente le résidu de formule : 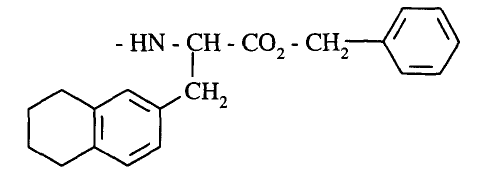
**Thn-OEt** représente le résidu de formule : 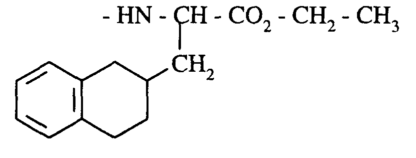
**Thi-OBzl(4-Cl)** représente le résidu de formule : 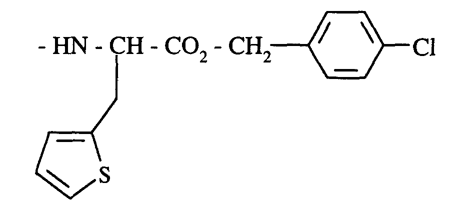
**Phe-OBzl(3,5-ditrifluorométhyl)** représente le résidu de formule : 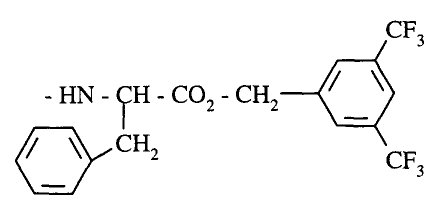
**Bza-OBzI** représente le résidu de formule : 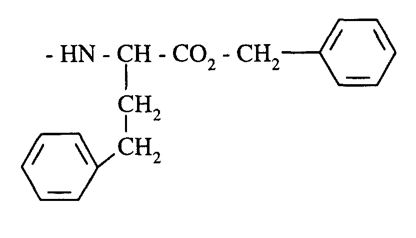
**Cha-OBzI** représente le résidu de formule : 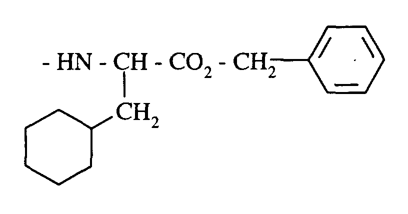
**Trp-OBzI(4-MeO)** représente le résidu de formule :

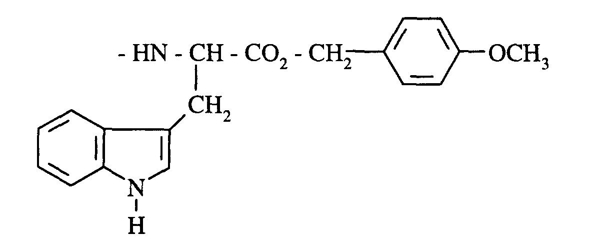

### Exemple 1: {1-[4-(1H-Tétrazol-5-yl)butyl]indol-3 -yl}carbonyl-Hyp-Trp-OBzl (3,5-ditrifluorométhyl), sel de potassium

### Stade A : Ester benzylique de l'acide 1-(4-cyanobutyl)indole-3-carboxylique

A 40 mmoles d'ester benzylique de l'acide indole-3-carboxylique, dissoutes dans du tétrahydrofurane, sont ajoutés 1,1 équivalents de 5-bromovaleronitrile. Après refroidissement à 0°C, 1,8 g d'hydrure de sodium sont ajoutés par fractions. Après une heure de réaction à 0°C et 14 heures à température ambiante, on ajoute de l'acétate d'éthyle et de l'eau. La phase organique est séparée et lavée plusieurs fois successivement par des phases aqueuses chlorhydrique puis basique. Après séchage et évaporation, le résidu est purifié par chromatographie sur gel de silice en utilisant comme solvant un mélange acétate d'éthyle/pentane (2/3) et on obtient le produit attendu.

### Stade B : Acide 1-(4-cyanobutyl)indole-3-carboxylique

10 mmoles du composé obtenu au stade précédent dissoutes dans 120 ml de méthanol sont hydrogénées à pression atmosphérique pendant 3 heures et demi en présence de 0,4 g de Pd/C comme catalyseur. Après filtration sur célite et évaporation, on obtient le produit attendu qui est recristallisé dans un mélange chloroforme/pentane.

### Stade C : [1-(4-Cyanobutyl)indol-3-yl]carbonyl-Hyp(tBu)-OBzl

A une solution contenant 10 mmoles de H-Hyp(tBu)-OBzl (obtenu selon le procédé décrit dans le brevet EP 0684257) dans 20 ml de dichlorométhane sont ajoutées 10 mmoles du produit obtenu au stade précédent, 15 mmoles de bromo-tris-pyrrolidino-phosphonium hexafluorophosphate et 20 mmoles de diisopropyléthylamine. Après 30 minutes à 0°C, le mélange est amené à température ambiante et maintenu 17 heures sous agitation. Après évaporation, l'huile résiduelle est reprise dans l'acétate d'éthyle. La phase organique est lavée avec des solutions aqueuses de carbonate de sodium, d'acide citrique et de chlorure de sodium. Le produit attendu est alors isolé par chromatographie sur gel de silice en utilisant comme éluant un mélange dichlorométhane/acétone (9/1).

### Stade D : [1-(4-Cyanobutyl)indol-3-yl]carbonyl-Hyp(tBu)-OH

8 mmoles du composé obtenu au stade précédent dans 20 ml de tétrahydrofurane sont traitées par 4 équivalents de soude pulvérisée et 20 mg de catalyseur tétrabutylammonium hydrogénosulfate pendant 16 heures à température ambiante sous agitation. La solution est alors acidifiée par 350 ml d'acide citrique à 5 %. Après extraction à l'acétate d'éthyle et lavage, le produit attendu est obtenu par précipitation du résidu dans du pentane.

### Stade E : [1-(4-Cyanobutyl)indol-3-yl]carbonyl-Hyp(tBu)-Trp-OBzI (3,5-ditrifluorométhyl)

7 mmoles du composé obtenu au stade précédent sont dissoutes dans 30 ml de dichlorométhane et couplées avec 7 mmoles de chlorhydrate de H-Trp-OBzl(3,5-ditrifluorométhyl) en présence de 7 mmoles de 1-hydroxybenzotriazole, de 7,7 mmoles de 2-(1H-benzotriazol-1-yl)-1,1,3,3-tétraméthyluronium tétrafluoroborate. Au mélange refroidi dans un bain de glace, sont ajoutées 15 mmoles de diisopropyléthylamine. L'ensemble est maintenu 30 minutes à 0°C et 14 heures à température ambiante. Après évaporation du solvant, le résidu est repris par de l'acétate d'éthyle. La phase organique est lavée et évaporée et le produit attendu est obtenu par précipitation dans l'éther.

### Stade F : {1-[4-(1H-Tétrazol-5-yl)butyl]indol-3-yl}carbonyl-Hyp(tBu)-Trp-OBzl (3,5-ditrifluorométhyl)

4 mmoles du composé obtenu au stade précédent en suspension dans 80 ml de toluène sont traitées successivement par 12 mmoles de triméthylazidosilane (TMSiN3) et 0,8 mmoles de dibutylétain. La suspension est agitée 20 heures à 80°C. Après évaporation du solvant, le résidu est repris par de l'acétate d'éthyle. Après lavage de la phase aqueuse, séchage puis évaporation, le résidu est purifié par chromatographie sur gel de silice en utilisant un mélange dichlorométhane/ méthanol (95/5) et conduit au produit attendu.

### Stade G : {1-[4-(1H-Tétrazol-5-yl)butyl]indol-3-yl}carbonyl-Hyp-Trp-OBzl (3,5-ditrifluorométhyl)

3 mmoles du produit obtenu au stade précédent dissoutes dans 100 ml d'un mélange acide trifluoroacétique/dichlorométhane (50/50) sont agitées 90 minutes à température ambiante. Après précipitation dans l'éther, le produit attendu est purifié par chromatographie liquide, préparative sur phase inverse C₁₈ en utilisant comme éluant un gradient de 40 à 60 % d'acétonitrile/eau - acide trifluoroacétique 0,1 %.

### Stade H : {1-[4-(1H-Tétrazol-5-yl)butyl]indol-3-yl}carbonyl-Hyp-Trp-OBzl (3,5-ditrifluorométhyl), sel de potassium

Le sel de potassium du produit obtenu au stade précédent est obtenu par addition de 2,2 mmoles de potasse en solution 0,1 N dans un mélange eau/acétonitrile (1/1) à une solution refroidie contenant 2 mmoles du composé du stade précédent. Le sel est alors lyophilisé.
Spectre de masse : FAB : [M+H]⁺ - m/z = 849

Les exemples suivants ont été obtenus selon le procédé décrit dans l'exemple 1 en utilisant les produits de départ correspondants :

### Exemple 2: {1-[4-(1H-Tétrazol-5-yl)butyl]indol-3-yl}carbonyl-Hyp-Nal-OBzl (3,5-ditrifluorométhyl), sel de potassium

### Exemple 3 : {1-[4-(1H-Tétrazol-5-yl)butyl]indol-3-yl}carbonyl-Hyp-Tna-OBzl

### Exemple 4: {1-[4-(1H-Tétrazol-5-yl)butyl}indol-3-yl}carbonyl-Hyp-Thn-OEt

### Exemple 5: {1-[(1H-Tétrazol-5-yl)méthyl]indol-3-yl}carbonyl-Hyp-Trp-OBzl (3,5-ditrifluorométhyl)

### Exemple 6: {1-[2-(1H-Tétrazol-5-yl)éthyl]indol-3-yl}carbonyl-Hyp-Trp-OBzl

### Exemple 7 : {1-[3-(1H-Tétrazol-5-yl)propyl]indol-3-yl}carbonyl-Hyp-Trp-OBzl

### Exemple 8: {1-[5-(1H-Tétrazol-5-yl)pentyl]indol-3-yl}carbonyl-Hyp-Trp-OBzl (3,5-ditrifluorométhyl)

### Exemple 9 : {1-[4-(1H-Tétrazol-5-yl)butyl]indol-3-yl}carbonyl-Hyp-Bza-OBzl

### Exemple 10: {1-[4-(1H-Tétrazol-5-yl)butyl]indol-3-yl}carbonyl-Hyp-Thi-OBzl(4-Cl)

### Exemple 11: {1-[4-(1H-Tétrazol-5-yl)bulyl]indol-3-yl}carbonyl-Hyp-Phe-OBzl (3,5-ditrifluorométhyl)

### Exemple 12: {1-[4-(1H-Tétrazol-5-yl)butyl]indol-3-yl}carbonyl-Hyp-Cha-OBzl

### Exemple 13: {1-[4-(1H-Tétrazol-5-yl)butyl]indol-3-yl}carbonyl-Hyp-Trp-OBzl(4-MeO)

### Etude pharmacologique des dérivés de l'invention

### Exemple 14 : Affinité aux récepteurs humains NK₁ et NK₂

L'affinité pour les récepteurs humains NK₁ et NK₂ a été étudiée sur les lymphoblastes humains IM-9 exprimant spécifiquement le récepteur NK₁ comme décrit par D.G. PAYAN et coll. (J. Biol. Chem. 1986, 261, 14321-14329) et sur les cellules CHO-K1 transfectées avec le récepteur NK₂ selon le kit "CellPhect transfection" (Pharmacia). Les composés de l'invention ont montré une excellente affinité spécifique pour les récepteurs NK₁. Le composé de l'exemple 1 en particulier présente un KI pour le récepteur NK₁ égal à 2,4 nM alors que celui pour le récepteur NK₂ est égal à 4200 nM.

### Exemple 15 : Essais sur le muscle lisse isolé

Afin d'évaluer l'activité fonctionnelle des composés de l'invention comme antagonistes des neurokinines, deux préparations isolées de muscle lisse ont été utilisées : la veine cave de lapin (VCL), l'artère pulmonaire de lapin sans endothélium (APL) dont les réponses contractiles sont respectivement médiées par les récepteurs NK₁ et NK₂ comme indiqué par D. JUKIC et coll. (Life Sci. 1991, 49, 1463-1469). Le pouvoir antagoniste des composés de l'invention a été exprimé sous forme de pA₂ tel que défini par O. ARUNLAKSHANA et H.O. SCHILD (Brit. J. Pharmacol. 1959, 14, 48-58).

Les composés de l'invention ont montré une puissante activité antagoniste vis-à-vis des récepteurs NK₁ avec une faible activité pour les récepteurs NK₂. Le composé de l'exemple 1 par exemple présente un pA₂ vis-à-vis des récepteurs NK₁ égal à 8,9 alors que son pA₂ vis-à-vis des récepteurs NK₂ est égal à 5,2.

### Exemple 16: Etude de l'inhibition de l'extravasation plasmatique induite par capsaicine chez le cobaye

L'effet des composés sur l'extravasation plasmatique provoquée par injection intraveineuse de capsaïcine (200 µg/kg) chez le cobaye a été évalué au niveau des bronches, selon la méthode décrite par Robineau et col. (Eur. J. Pharmacol., 1995, 294, 677-684). L'accumulation au niveau bronchique de bleu Evan's injecté IV 1 minute avant la capsaicine a été quantifiée par spectrophotométrie après extraction du colorant par l'acétone. L'activité inhibitrice des composés par voie intraveineuse 5 minutes avant la capsaicine a été exprimée en dose inhibitrice 50 % par comparaison à un lot contrôle (8 animaux par lot). Le composé de l'exemple 1 possède une ED₅₀ de 26 µg/kg iv alors que le composé de l'exemple 1 du brevet EP 684 257 présente une ED₅₀ de 140 µg/kg soit environ 6 fois moins importante.

### Exemple 17: Etude de l'inhibition de la bronchoconstriction induite par la substance P chez le cobaye

L'étude est réalisée sur des cobayes Hartley mâles (Charles River) de poids moyen 300 à 400g. L'étude est réalisée sur des animaux anesthésiés (carbamate d'éthyle 1,5 g/kg) et curarisés flaxedil (0,2 mg/kg iv) ventilés avec une fréquence de 60 par minute et un volume courant de 10 ml/kg. Les animaux sont pré-traités par pyrilamine (1 mg/kg iv), propranolol (1 mg/kg iv).
Le critère de jugement de la bronchoconstriction est l'augmentation de la pression d'insuflation trachéale (PIT) induite par l'injection de substance P par voie iv à la dose de 2 nM/kg, iv, chaque animal étant son propre témoin. L'injection du produit testé s'effectuant par rapport au temps T0 injection du produit.

Les résultats sont exprimés en dose inhibitrice 50 % de la bronchoconstriction induite par la substance P, ce pourcentage étant calculé selon la formule suivante :
(Δ PIT avant produit - Δ PIT après produit) / Δ PIT avant produit (exprimé en pourcentage).
Le composé de l'exemple 1 présente une ED₅₀ égale à 0,06 mg/kg. Il est deux fois et demi plus puissant que le composé de l'exemple 1 du brevet EP 684 257.

### Exemple 18: Etude de la dégranulation mastocytaire in vitro

Les études sont effectuées sur mastocytes péritonéaux de rats mâles : Sprague-Dawley de poids moyen 350 à 400 g et sont euthanasiés par inhalation de CO₂. Un lavage péritonéal est effectué avec 20 ml de tampon (NaCI 150 mM, KCl 2.7 mM, CaCl₂, 0.9 mM, Na₂HPO₄, 3 mM, KH₂PO₄, 3.5 mM, glucose 5.6 mM, albumine de sérum bovin 1 mg/ml, pH 6.8).
Le liquide de lavage est centrifugé à 400 g pendant 10 mn à 4°C et le culot repris dans un tampon à une densité de 2.10⁴ mastocytes/ml. Les composés sont mis à incuber à concentration croissante pendant 20 mn. La réaction est stoppée en plaçant les tubes sur glace et une centrifugation à 700 g pendant 10 mn à 4°C est effectuée. Le surnageant et le culot sont testés par fluorimétrie après condensation avec O-phtalaldehyde. L'histamine libérée à partir des cellules dans le surnageant ainsi dosée est exprimée en pourcentage du contenu total d'histamine des cellules. Lors de ce test, les composés de l'invention n'entraînent pas de dégranulation mastocytaire jusqu'à la concentration de 10⁻⁴M.

### Composition pharmaceutique

### EXEMPLE 19 : Comprimé : formule de préparation pour 1000 comprimés dosés à 2 mg

| | |
|---|---|
| Composé de l'exemple 1 | 2 g |
| Hydroxypropylcellulose | 2 g |
| Amidon de blé | 10 g |
| Lactose | 100 g |
| Stéarate de magnésium | 3 g |
| Talc | 3 g |

## Revendications

1. Composé de formule (I) : 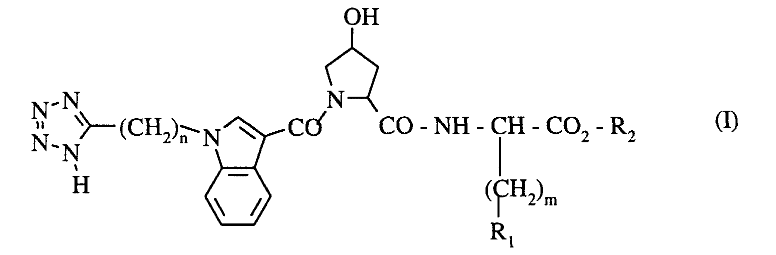 dans laquelle :
R₁ représente un groupement indolyle, naphtyle, tétrahydronaphtyle, thiényle, phényle (éventuellement substitué par un ou plusieurs atomes d'halogène ou groupements alkyle (C₁-C₆) linéaire ou ramifié, alkoxy (C₁-C₆) linéaire ou ramifié, hydroxy, trihalogénométhyle) ou cycloalkyle (C₃-C₇),
n représente un entier tel que 1 ≤ n ≤ 8,
m représente un entier tel que 1 ≤ m ≤ 4,
R₂ représente un groupement benzyle (substitué ou non par un ou plusieurs atomes d'halogène ou groupements alkyle (C₁-C₆) linéaire ou ramifié, alkoxy (C₁-C₆) linéaire ou ramifié, hydroxy ou trihalogénométhyle), un groupement phényle (substitué ou non par un ou plusieurs atomes d'halogène ou groupements alkyle (C₁-C₆) linéaire ou ramifié, alkoxy (C₁-C₆) linéaire ou ramifié, hydroxy ou trihalogénométhyle), un groupement alkyle (C₁-C₆) linéaire ou ramifié,
leurs énantiomères, diastéréoisomères et épimères ainsi que leurs sels d'addition à un acide ou à une base pharmaceutiquement acceptable.

2. Composé de formule (I) selon la revendication 1 tels que R₁ représente un groupement indolyle.

3. Composé de formule (I) selon la revendication 1 tels que R₂ représente un groupement benzyle substitué ou non.

4. Composé de formule (I) selon l'une quelconque des revendications 1 ou 3 tels que R₂ représente un groupement 3,5-di(trifluorométhyl)benzyle.

5. Composé de formule (I) selon la revendication 1 tels que n est égal à 4.

6. Composé de formule (I) selon la revendication 1 tels que m est égal à 1.

7. Composé de formule (I) selon l'une quelconque des revendications 1, 2, 3, 4,5 ou 6 qui est le {1-[4-(1H-tétrazol-5-yl)butyl]indol-3-yl}carbonyl-Hyp-Trp-OBzl(3,5-ditrifluorométhyl), ses isomères ainsi que ses sels d'addition à un acide ou à une base pharmaceutiquement acceptable.

8. Procédé de préparation des composés de formule (I) selon la revendication 1 caractérisé en ce que l'on fait réagir une hydroxyproline protégée, de formule (II), dont on a éventuellement séparé les isomères : 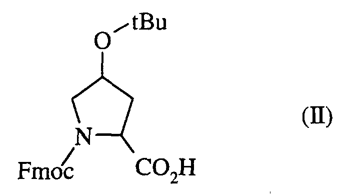 dans laquelle :
Fmoc représente le groupement 9-fluorénylméthoxycarbonyle,
tBu représente le groupement tert-butyle,
avec de l'alcool benzylique, en présence de 4-diméthylaminopyridine et de N,N-dicyclohexylcarbodiimide,
pour conduire au composé de formule (III) : 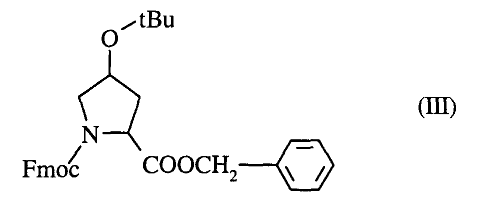 dans laquelle Fmoc et tBu ont les mêmes significations que précédemment,
dont on déprotège la fonction amine en milieu pipéridine,
pour conduire au composé de formule (IV) : 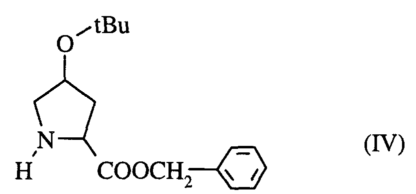 dans laquelle tBu a la même signification que précédemment,
que l'on fait réagir avec de l'acide indole-3-carboxylique,
pour conduire au composé de formule (V) : 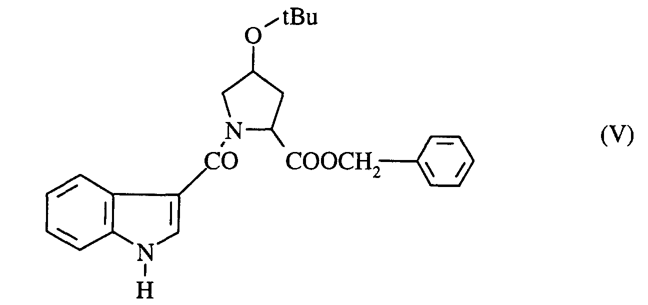 dans laquelle tBu a la même signification que précédemment,
dont on déprotège la fonction acide carboxylique, par hydrogénation catalytique,
pour conduire au composé de formule (VI) : 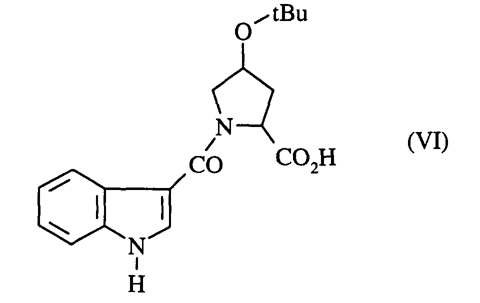 dans laquelle tBu a la même signification que précédemment, que l'on met en réaction avec un amino-acide protégé de formule (VII), dont on a éventuellement séparé les isomères : 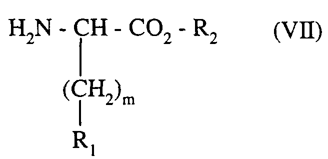 dans laquelle R₁, R₂ et m ont la même signification que dans la formule (I),
pour conduire au composé de formule (VIII) : 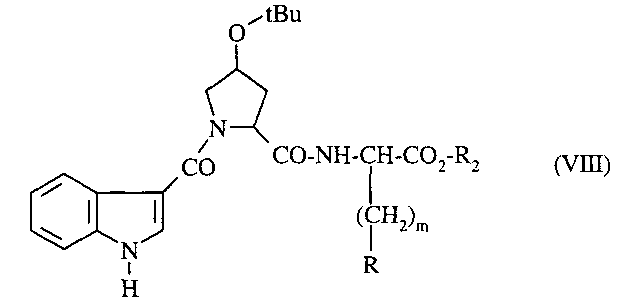 dans laquelle tBu, R₁, R₂ et m ont la même signification que précédemment,
que l'on fait réagir sur un tétrazole protégé de formule (IX), en présence d'hydrogénosulfate de tétrabutylammonium, 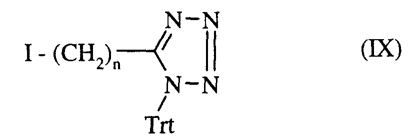 dans laquelle Trt représente le groupement triphénylméthyle,
pour conduire au composé de formule (X) : 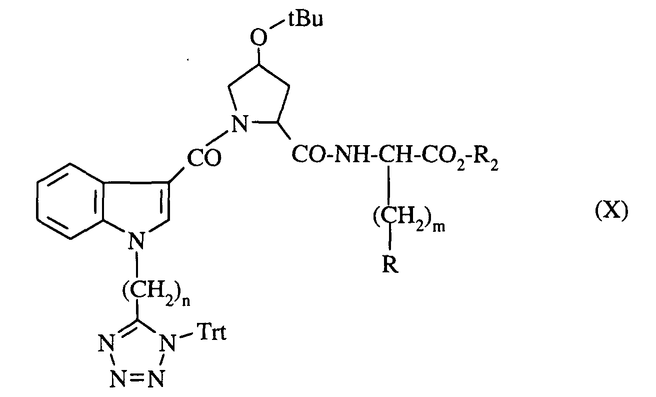 dans laquelle tBu, R₁, R₂, m, n et Trt ont la même signification que précédemment, que l'on déprotège, en milieu acide trifluoroacétique,
pour conduire au composé de formule (I),
- qui peut être, le cas échéant, purifié selon une technique classique de purification,
- dont on sépare, le cas échéant, les isomères selon une technique classique de séparation,
- que l'on transforme, si on le souhaite, en ses sels d'addition à une base pharmaceutiquement acceptable.

9. Procédé de préparation des composés de formule (I) selon la revendication 1 caractérisé e ce que l'on fait réagir l'ester benzylique de l'acide indole-3-carboxylique avec u bromonitrile de formule (XIV) en présence d'hydrure de sodium :
Br - (CH₂)ₙ - CN (XIV)
dans laquelle n a la même signification que dans la formule (I),
pour conduire au composé de formule (XV) : 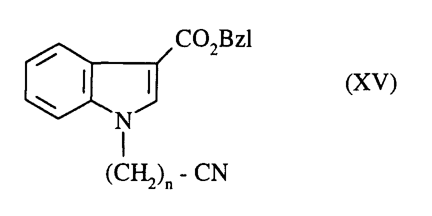 dans laquelle n a la même signification que dans la formule (I) et Bzl représente un groupement benzyle, dont on déprotège la fonction acide par hydrogénation catalytique,
pour conduire au composé de formule (XVI) : 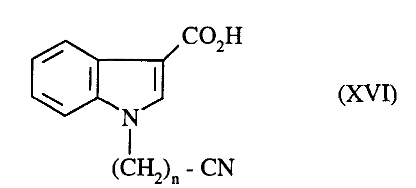 dans laquelle n a la même signification que dans la formule (I), que l'on fait réagir avec une hydroxyproline protégée de formule (XVII), en présence de bromo-tris-pyrrolidino-phosphonium hexafluorophosphate et de diisopropyléthylamine : 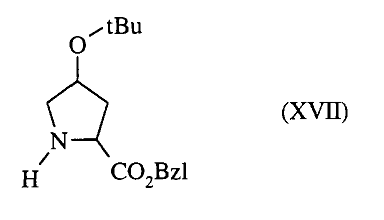 dans laquelle tBu représente le groupement tert-butyle et Bzl le groupement benzyle, pour conduire au composé de formule (XVIII) : 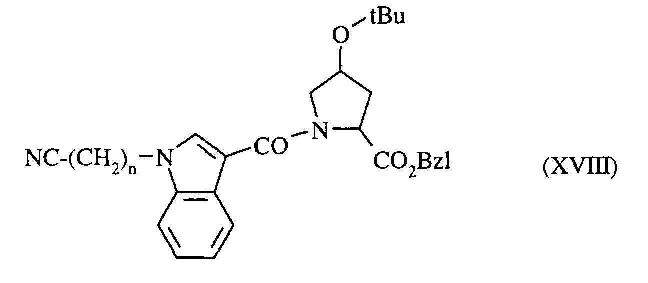 dans laquelle n, tBu et Bzl ont la même signification que précédemment, dont on déprotège la fonction acide en milieu basique en présence d'un catalyseur,
pour conduire au composé de formule (XIX) : 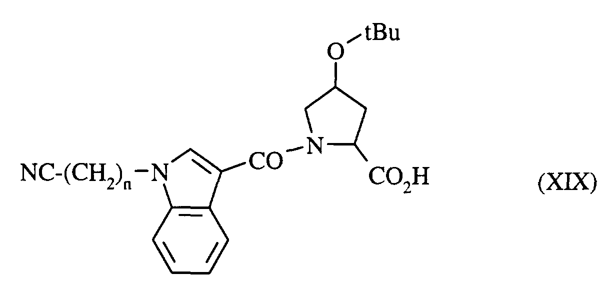 dans laquelle n et tBu ont la même signification que précédemment, que l'on fait réagir avec un composé de formule (XX) en présence d'un réactif de couplage peptidique : 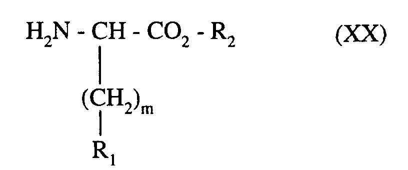 dans laquelle R₁, R₂ et m ont la même signification que dans la formule (I),
pour conduire au composé de formule (XXI) : 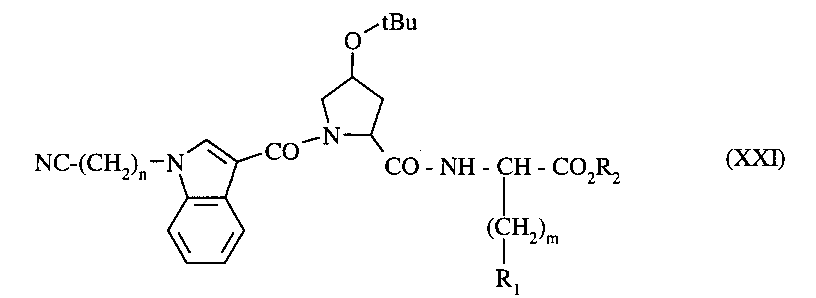 dans laquelle R₁, R₂, n, m et tBu ont la même signification que précédemment, que l'on traite ensuite avec du triméthylazidosilane et de l'oxyde de dibutylétain,
pour conduire au composé de formule (XXII) : 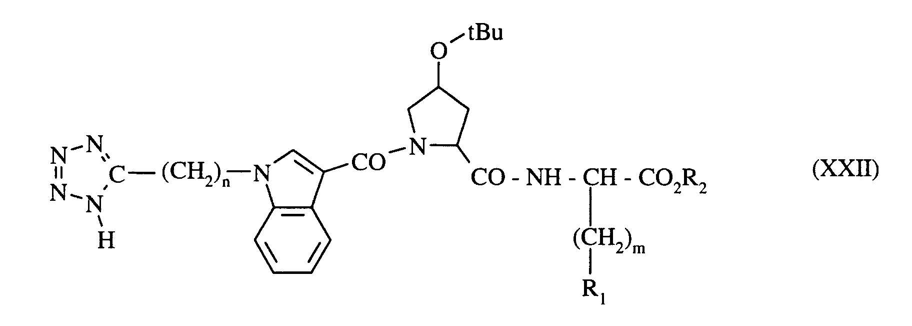 dans laquelle R₁, R₂, n, m et tBu ont la même signification que précédemment, que l'on déprotège en milieu acide trifluoroacétique, pour conduire au composé de formule (I),
- qui peut être, le cas échéant, purifié selon une technique classique de purification,
- dont on sépare, le cas échéant, les isomères selon une technique classique de séparation,
- que l'on transforme, si on le souhaite, en ses sels d'addition à une base pharmaceutiquement acceptable.

10. Compositions pharmaceutiques contenant comme principe actif au moins un composé selon l'une quelconque des revendications1 à 7, seul ou en combinaison avec un ou plusieurs excipients ou véhicules inertes, non toxiques, pharmaceutiquement acceptables.

11. Compositions pharmaceutiques selon la revendication 10 contenant au moins un principe actif selon l'une quelconque des revendications 1 à 7 utiles comme antagonistes de substance P dans le traitement de la douleur, de l'inflammation, des troubles gastro-intestinaux ou urinaires, de l'asthme, des bronchopathies chroniques, des allergies, de la migraine, des maladies de système nerveux central ainsi qu'en tant qu'antiémétiques.

## Patentansprüche

1. Verbindung der Formel (I): 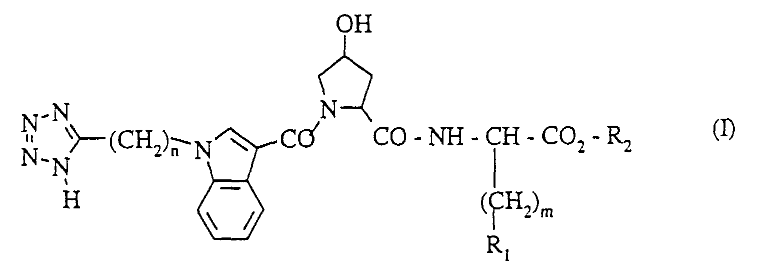 in der:
R₁ eine Indolyl-, Naphthyl-, Tetrahydronaphthyl-, Thienyl- oder Phenylgruppe (die gegebenenfalls durch ein oder mehrere Halogenatome, geradkettige oder verzweigte (C₁-C₆)-Alkylgruppen, geradkettige oder verzweigte (C₁-C₆)-Alkoxygruppen, Hydroxygruppen oder Trihalogenmethylgruppen substituiert sind) oder eine (C₃-C₇)-Cycloalkylgruppe darstellt,
n eine ganze Zahl gemäß der folgenden Beziehung 1 ≤ n ≤ 8 darstellt,
m eine ganze Zahl gemäß der folgenden Beziehung 1 ≤ m ≤ 4 darstellt,
R2 eine Benzylgruppe (die gegebenenfalls durch ein oder mehrere Halogenatome, geradkettige oder verzweigte (C₁-C₆)-Alkylgruppen, geradkettige oder verzweigte (C₁-C₆)-Alkoxygruppen, Hydroxygruppen oder Trihalogenmethylgruppen substituiert ist), eine Phenylgruppe (die gegebenenfalls durch ein oder mehrere Halogenatome, geradkettige oder verzweigte (C₁-C₆)-Alkylgruppen, geradkettige oder verzweigte (C₁-C₆)-Alkoxygruppen, Hydroxygruppen oder Trihalogenmethylgruppen substituiert ist) oder eine geradkettige oder verzweigte (C₁-C₆)-Alkylgruppe bedeutet,
deren Enantiomere, Diastereoisomere und Epimere sowie deren Additionssalze mit einer pharmazeutisch annehmbaren Säure oder Base.

2. Verbindung der Formel (I) nach Anspruch 1, worin R₁ eine Indolylgruppe darstellt.

3. Verbindung der Formel (I) nach Anspruch 1, worin R₂ eine gegebenenfalls substituierte Benzylgruppe darstellt.

4. Verbindung der Formel (I) nach einem der Ansprüche 1 oder 3, worin R₂ eine 3,5-Di-(trifluormethyl)-benzylgruppe darstellt.

5. Verbindung der Formel (I) nach Anspruch 1, worin n den Wert 4 besitzt.

6. Verbindung der Formel (I) nach Anspruch 1, worin m den Wert 1 besitzt.

7. Verbindung der Formel (I) nach einem der Ansprüche 1, 2, 3, 4, 5 oder 6, nämlich {1-[4(1H-Tetrazol-5-yl)-butyl]-indol-3-yl}-carbonyl-Hyp-Trp-OBzl-(3,5-ditrifluormethyl), dessen Isomere sowie dessen Additionssalze mit einer pharmazeutisch annehmbaren Säure oder Base.

8. Verfahren zur Herstellung der Verbindungen der Formel (I) nach Anspruch 1, dadurch gekennzeichnet, daß man ein geschütztes Hydroxyprolin der Formel (II), welches man gegebenenfalls in seine Isomeren aufgetrennt hat: 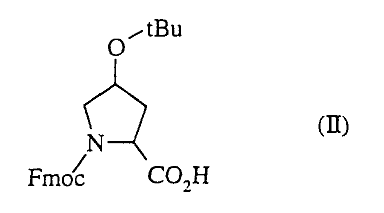 in der:
Fmoc die 9-Fluorenylmethoxycarbonylgruppe und
tBu die tert.-Butylgruppe bedeuten,
mit Benzylalkohol in Gegenwart von 4-Dimethylaminopyridin und N,N-Dicyclohexylcarbodiimid umsetzt,
zur Bildung der Verbindung der Formel (III): 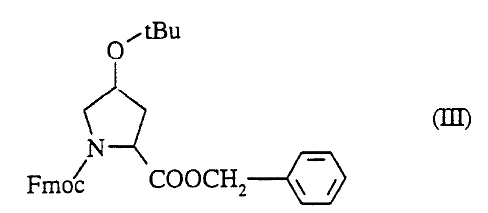 in der Fmoc und tBu die oben angegebenen Bedeutungen besitzen,
von der man die Schutzgruppe der Aminogruppe in Piperidinmedium abspaltet,
zur Bildung der Verbindung der Formel (IV): 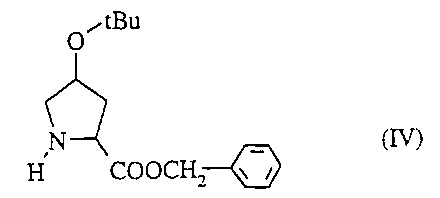 in der tBu die oben angegebenen Bedeutungen besitzt,
welche man mit Indol-3-carbonsäure umsetzt,
zur Bildung der Verbindung der Formel (V): 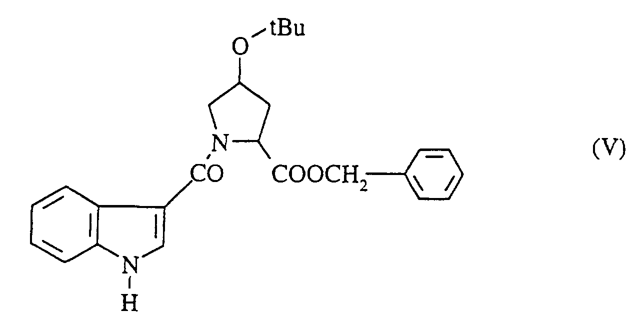 in der tBu die oben angegebenen Bedeutungen besitzt,
von der man die Schutzgruppe der Carbonsäurefunktion durch katalytische Hydrierung abspaltet,
zur Bildung der Verbindung der Formel (VI): 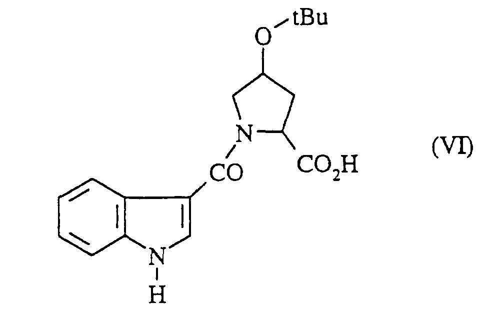 in der tBu die oben angegebenen Bedeutungen besitzt,
welche man mit einer geschützten Aminosäure der Formel (VII), die man gegebenenfalls in ihre Isomeren aufgespalten hat: 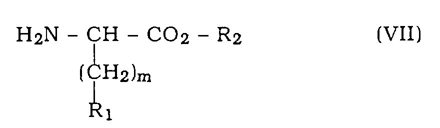 in der R₁, R₂ und m die bezüglich der Formel (I) angegebenen Bedeutungen besitzen, umsetzt,
zur Bildung der Verbindung der Formel (VIII): 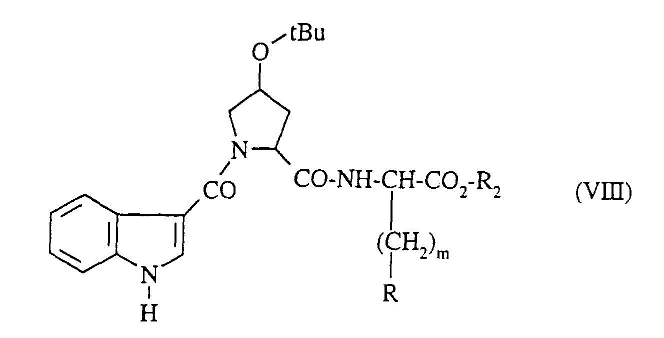 in der tBu, R₁, R₂ und m die oben angegebenen Bedeutungen besitzen,
welche man in Gegenwart von Tetrabutylammoniumhydrogensulfat mit einem geschützten Tetrazol der Formel (IX): 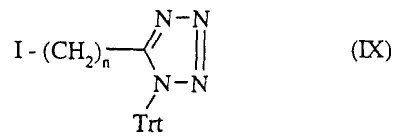 in der Trt die Triphenylmethylgruppe bedeutet, umsetzt,
zur Bildung der Verbindung der Formel (X): 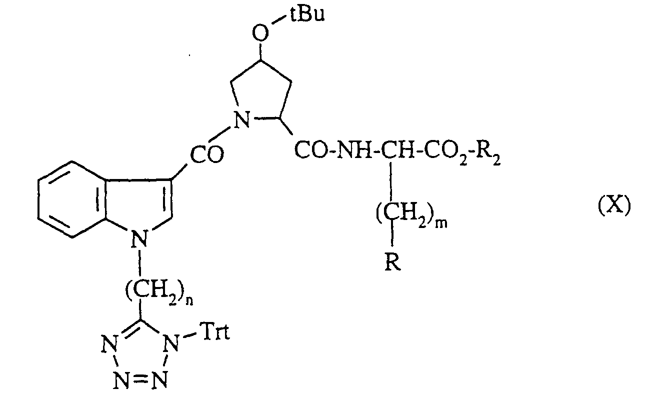 in der tBu, R₁, R₂, m, n und Trt die oben angegebenen Bedeutungen besitzen,
welche man in trifluoressigsaurem Medium von der Schutzgruppe befreit,
zur Bildung der Verbindung der Formel (I),
- welche man gegebenenfalls mit Hilfe einer klassischen Reinigungsmethode reinigen kann,
- welche man gegebenenfalls mit Hilfe einer klassischen Trennmethode in ihre Isomeren auftrennen kann
- und welche man gewünschtenfalls in ihre Additionssalze mit einer pharmazeutisch annehmbaren Base umwandeln kann.

9. Verfahren zur Herstellung der Verbindungen der Formel (I) nach Anspruch 1, dadurch gekennzeichnet, daß man den Benzylester der Indol-3-carbonsäure in Gegenwart von Natriumhydrid mit einem Bromnitril der Formel (XIV):
Br - (CH₂)ₙ - CN (XIV)
in der n die bezüglich der Formel (I) angegebenen Bedeutungen besitzt, umsetzt, zur Bildung der Verbindung der Formel (XV): 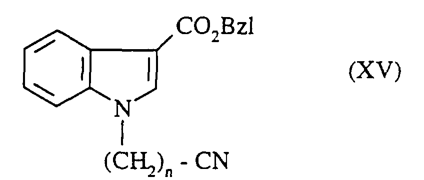 in der n die bezüglich der Formel (I) angegebenen Bedeutungen besitzt und Bzl für eine Benzylgruppe steht, von der man die Schutzgruppe der Säurefunktion durch katalytische Hydrierung abspaltet,
zur Bildung der Verbindung der Formel (XVI): 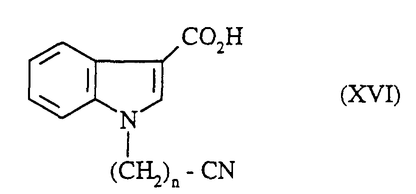 in der n die bezüglich der Formel (I) angegebenen Bedeutungen besitzt, welche man in Gegenwart von Brom-tris-pyrrolidinophosphoniumhexafluorphosphat und Diisopropylethylamin mit einem geschützten Hydroxyprolin der Formel (XVII): 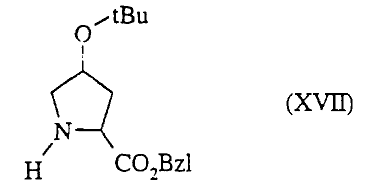 in der tBu für die tert.-Butylgruppe und Bzl für die Benzylgruppe stehen, umsetzt,
zur Bildung der Verbindung der Formel (XVIII): 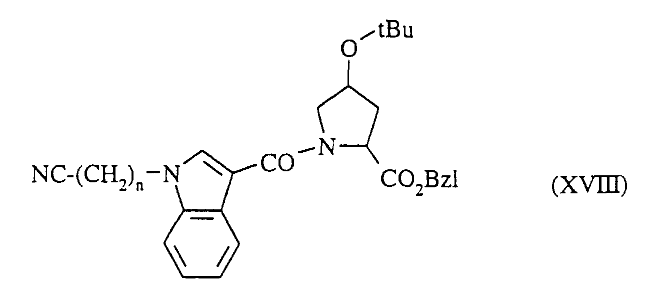 in der n, tBu und Bzl die oben angegebenen Bedeutungen besitzen, von welcher man die Schutzgruppe der Säurefunktion in basischem Medium in Gegenwart eines Katalysators abspaltet,
zur Bildung der Verbindung der Formel (XIX): 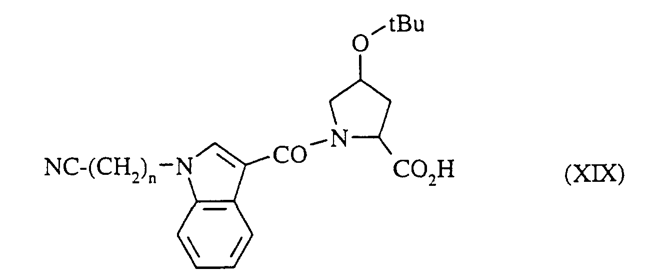 in der n und tBu die oben angegebenen Bedeutungen besitzen, welche man in Gegenwart eines Peptid-Kupplungsreagens mit einer Verbindung der Formel (XX) umsetzt: 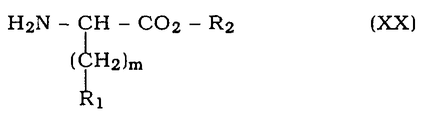 in der R₁, R₂ und m die bezüglich der Formel (I) angegebenen Bedeutungen besitzen,
zur Bildung der Verbindung der Formel (XXI): 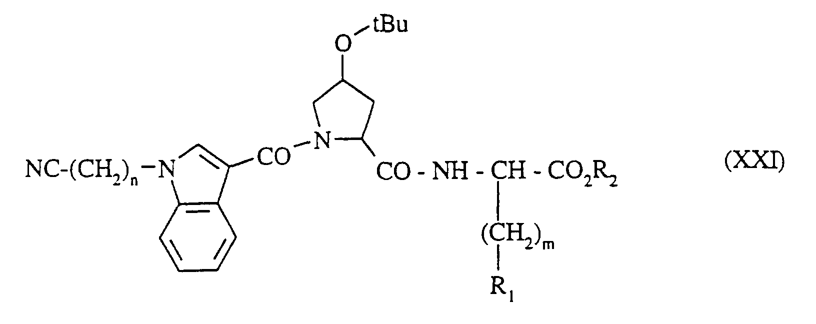 in der R₁, R₂, n, m und tBu die oben angegebenen Bedeutungen besitzen, welche man anschließend mit Trimethylazidosilan und Dibutylzinnoxid behandelt,
zur Bildung der Verbindung der Formel (XXII): 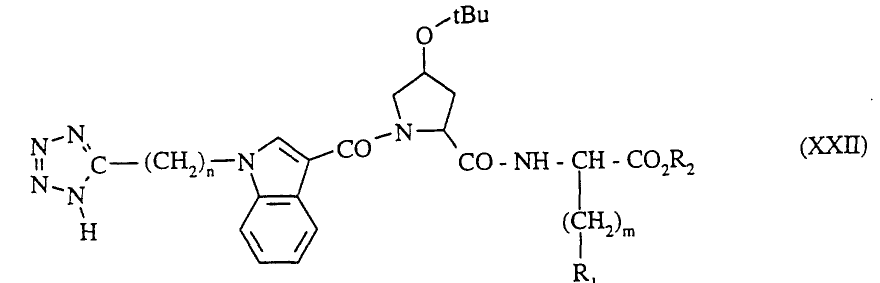 in der R₁, R₂, n, m und tBu die oben angegebenen Bedeutungen besitzen, von welcher man in trifluoressigsaurem Medium die Schutzgruppe abspaltet, zur Bildung der Verbindung der Formel (I),
- welche man gegebenenfalls mit Hilfe einer klassischen Reinigungsmethode reinigen kann,
- welche man gegebenenfalls mit Hilfe einer klassischen Trennmethode in ihre Isomeren auftrennen kann,
- und welche man gewünschtenfalls in ihre Additionssalze mit einer pharmazeutisch annehmbaren Base umwandeln kann.

10. Pharmazeutische Zubereitungen enthaltend als Wirkstoff mindestens eine Verbindung nach einem der Ansprüche 1 bis 7 allein oder in Kombination mit einem oder mehreren inerten, nichttoxischen, pharmazeutisch annehmbaren Trägermaterialien oder Bindemitteln.

11. Pharmazeutische Zubereitungen nach Anspruch 10 enthaltend mindestens einen Wirkstoff nach einem der Ansprüche 1 bis 7 als Antagonisten der Substanz P bei der Behandlung von Schmerzen, Magen-Darm- oder Harnstörungen, Asthma, chronischen Bronchopathien, Allergien, der Migräne, von Erkrankungen des Zentralnervensystems sowie als Antiemetika.

## Claims

1. Compound of formula (I): 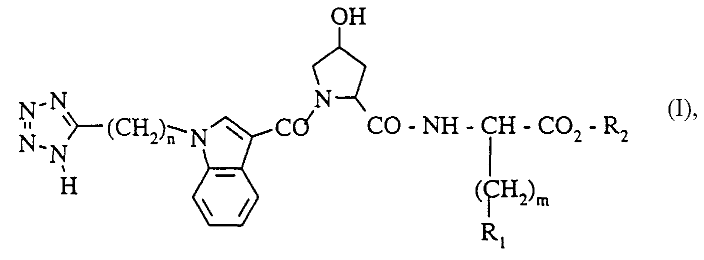 wherein :
R₁ represents an indolyl group, naphthyl group, tetrahydronaphthyl group, thienyl group, phenyl group (optionally substituted by one or more halogen atoms or linear or branched (C₁-C₆)alkyl, linear or branched (C₁-C₆)alkoxy, hydroxy or trihalomethyl groups) or (C₃-C₇)cycloalkyl group,
n represents an integer such that 1 ≤ n ≤ 8,
m 1 represents an integer such that 1 ≤ m ≤ 4,
R₂ represents a benzyl group (unsubstituted or substituted by one or more halogen atoms or linear or branched (C₁-C₆)alkyl, linear or branched (C₁-C₆)alkoxy, hydroxy or trihalomethyl groups), a phenyl group (unsubstituted or substituted by one or more halogen atoms or linear or branched (C₁-C₆)alkyl, linear or branched (C₁-C₆)alkoxy, hydroxy or trihalomethyl groups), or a linear or branched (C₁-C₆)alkyl group,
its enantiomers, diastereoisomers and epimers and addition salts thereof with a pharmaceutically acceptable acid or base.

2. Compound of formula (I) according to claim 1, wherein R₁ represents an indolyl group.

3. Compound of formula (I) according to claim 1, wherein R₂ represents an unsubstituted or substituted benzyl group.

4. Compound of formula (I) according to either of claims 1 or 3, wherein R₂ represents a 3,5-di(trifluoromethyl)benzyl group.

5. Compound of formula (I) according to claim 1, wherein n is 4.

6. Compound of formula (I) according to claim 1, wherein m is 1.

7. Compound of formula (I) according to any one of claims 1, 2, 3, 4, 5 or 6, which is {1-[4-(1H-tetrazol-5-yl)butyl]indol-3-yl}carbonyl-Hyp-Trp-OBzl(3,5-ditrifluoromethyl), its isomers and addition salts thereof with a pharmaceutically acceptable acid or base.

8. Process for the preparation of compounds of formula (I) according to claim 1, characterised in that a protected hydroxyproline of formula (II), optionally having been separated into its isomers : 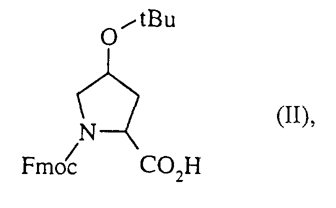 wherein:
Fmoc represents the group 9-fluorenylmethoxycarbonyl,
tBu represents the group tert-butyl,
is reacted with benzyl alcohol, in the presence of 4-dimethylaminopyridine and N,N-dicyclohexylcarbodiimide,
to yield the compound of formula (III) : 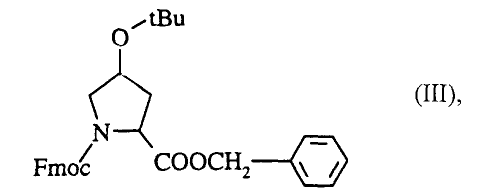 wherein Fmoc and tBu are as defined hereinbefore,
the amine function of which is deprotected in a piperidine medium
to yield the compound of formula (IV) : 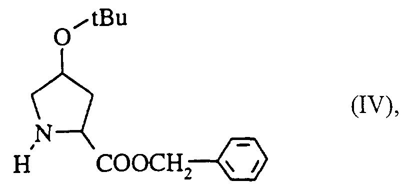 wherein tBu is as defined hereinbefore,
which is reacted with indole-3-carboxylic acid
to yield the compound of formula (V) : 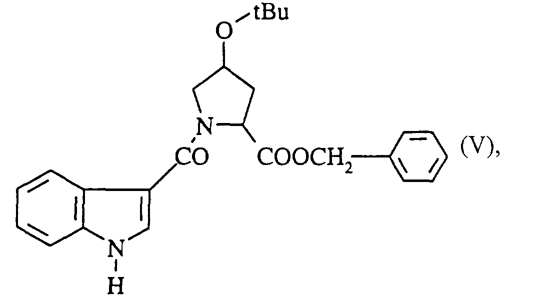 wherein tBu is as defined hereinbefore,
the carboxylic acid function of which is deprotected, by catalytic hydrogenation, to yield the compound of formula (VI) : 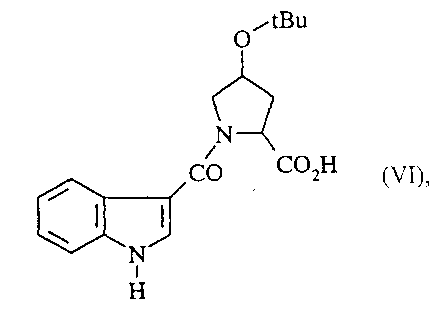 wherein tBu is as defined hereinbefore,
which is reacted with a protected amino acid of formula (VII), optionally having been separated into its isomers : 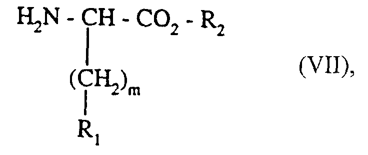 wherein R₁, R₂ and m are as defined for formula (I),
to yield the compound of formula (VIII): 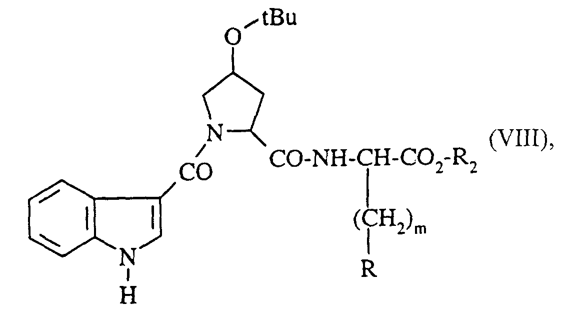 wherein tBu, R₁, R₂ and m are as defined hereinbefore,
which is reacted, in the presence of tetrabutylammonium hydrogen sulphate, with a protected tetrazole of formula (IX): 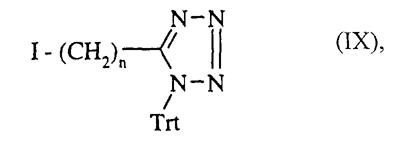 wherein Trt represents the group triphenylmethyl,
to yield the compound of formula (X): 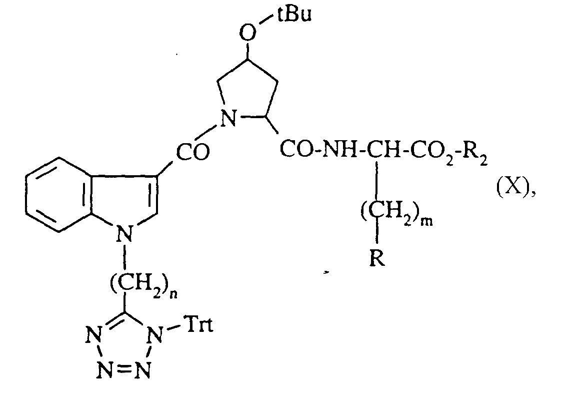 wherein tBu, R₁, R₂, m, n and Trt are as defined hereinbefore,
which is deprotected in a trifluoroacetic acid medium
to yield the compound of formula (I),
- which can be purified, where appropriate, according to a conventional purification technique,
- which is separated, where appropriate, into its isomers according to a conventional separation technique,
- which is converted, if desired, into its addition salts with a pharmaceutically acceptable base.

9. Process for the preparation of compounds of formula (I) according to claim 1, characterised in that indole-3-carboxylic acid benzyl ester is reacted, in the presence of sodium hydride, with a bromonitrile of formula (XIV) :
Br - (CH₂)ₙ - CN (XIV),
wherein n is as defined for formula (I),
to yield the compound of formula (XV) : 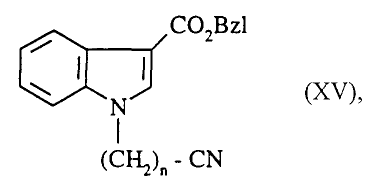 wherein n is as defined for formula (I) and Bzl represents a benzyl group, the acid function of which is deprotected by catalytic hydrogenation
to yield the compound of formula (XVI) : 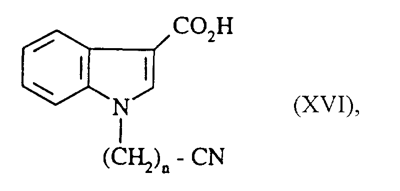 wherein n is as defined for formula (I), which is reacted, in the presence of bromo-tri pyrrolidino-phosphonium hexafluorophosphate and diisopropylethylamine, with protected hydroxyproline of formula (XVII) : 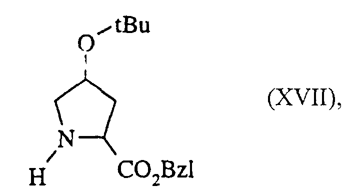 wherein tBu represents the group tert-butyl and Bzl represents the group benzyl, to yield the compound of formula (XVIII): 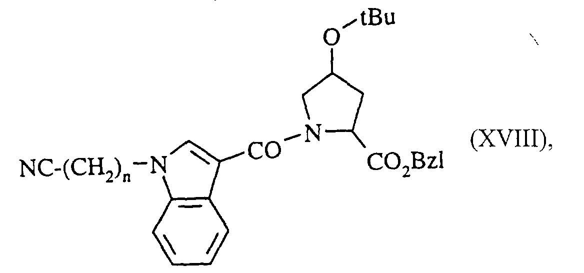 wherein n, tBu and Bzl are as defined hereinbefore, the acid function of which is deprotected in a basic medium in the presence of a catalyst
to yield the compound of formula (XIX): 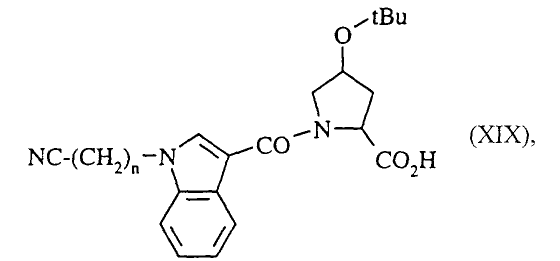 wherein n and tBu are as defined hereinbefore, which is reacted, in the presence of a peptide coupling reagent, with a compound of formula (XX): 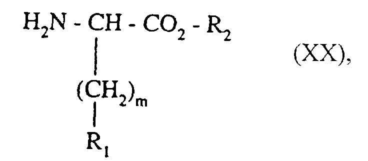 wherein R₁, R₂ and m are as defined for formula (I),
to yield the compound of formula (XXI): 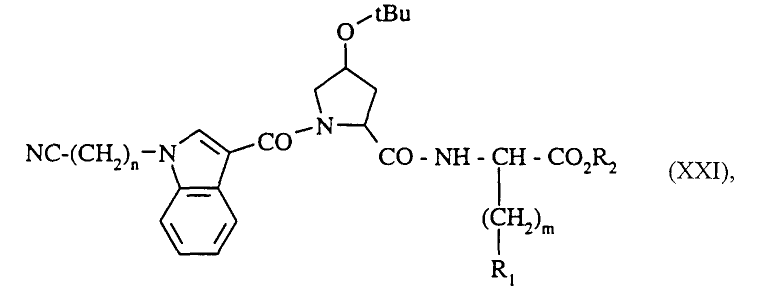 wherein R₁, R₂, n, m and tBu are as defined hereinbefore, which is then treated with trimethylazidosilane and dibutyltin oxide,
to yield the compound of formula (XXII): 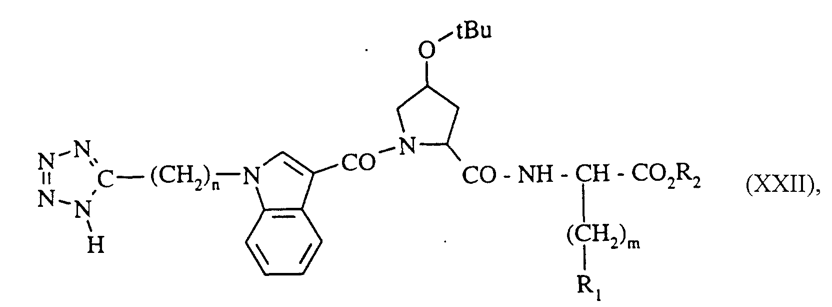 wherein R₁, R₂, n, m and tBu are as defined hereinbefore, which is deprotected in a trifluoroacetic acid medium to yield the compound of formula (I),
- which can be purified, where appropriate, according to a conventional purification technique,
- which is separated, where appropriate, into its isomers according to a conventional separation technique,
- which is converted, if desired, into its addition salts with a pharmaceutically acceptable base.

10. Pharmaceutical compositions comprising as active ingredient at least one compound according to any one of claims 1 to 7, alone or in combination with one or more pharmaceutically acceptable, inert, non-toxic excipients or carriers.

11. Pharmaceutical compositions according to claim 10 comprising at least one active ingredient according to any one of claims 1 to 7, for use as antagonists of substance P in the treatment of pain, inflammation, gastrointestinal or urinary disorders, asthma, chronic broncopathies, allergies, migraine and diseases of the central nervous system and also as anti-emetics.
